# EUROPEAN PATENT APPLICATION

(11) **EP 2 711 013 A1**
(43) Date of publication of application: **26.03.2014**
(21) Application number: 12784911.5
(22) Date of filing: 17.05.2012
(51) Int. Cl.: A61K 35/74, A23K 1/16, A23K 1/18, A61P 37/04

(54) **IMMUNOSTIMULANT FOR ANIMALS, FEED CONTAINING SAME, AND METHOD FOR MANUFACTURING SAME**

(30) Priority: 18.05.2011 JP 2011111503
(71) Applicant: Ajinomoto Co., Inc., Tokyo 104-8315 (JP)
(72) Inventor: TOKURA, Mitsunori, Kawasaki-shi, Kanagawa 210-8681 (JP); FUDO, Ryosuke, Kawasaki-shi, Kanagawa 210-8681 (JP); ONISHI, Norimasa, Kawasaki-shi, Kanagawa 210-8681 (JP); EBISAWA, Makoto, Kawasaki-shi, Kanagawa 210-8681 (JP); KOJIMA, Junichiro, Kawasaki-shi, Kanagawa 210-8681 (JP); OOTA, Yasuhiro, Kawasaki-shi, Kanagawa 210-8681 (JP); FUJIEDA, Takeshi, Kawasaki-shi, Kanagawa 210-8681 (JP)
(74) Representative: Strehl Schübel-Hopf & Partner
(86) International application number: PCT/JP2012/062634
(87) International publication number: WO 2012/157699

(57) **Abstract**

By administering sterilized bacterial cells obtained by heat treating cells of a coryneform bacterium or an enterobacterium under a condition of pH 2 to 5 at 90 to 120°C for 3 minutes to 4 hours, or a feed containing such sterilized bacterial cells to a mammal, fowl, fish, or crustacean, immunity of these animals is enhanced.

## Description

### Technical Field

The present invention relates to an immunostimulant used for mammals such as livestock animals and pet animals, birds such as domestic fowls, fishes such as farmed fishes, or crustaceans, a feed containing the immunostimulant, and a method for producing the immunostimulant, as well as a method for immunostimulation in these organisms.

### Background Art

In the stock raising industry and fisheries, infectious diseases caused by bacteria or viruses spread and prevail every year. In recent years, livestock and farmed fish productions are conducted in increasingly larger scales and more intensively. Therefore, they are bred in a high-density environment in limited facilities, and it is said that there are many individuals under severe stress and hypoimmune function state. Thus, once infection takes place, which results in herd infection, and causes serious economical damage. Specific examples of such diseases include porcine colibacillosis, porcine salmonellosis, bovine foot and mouth disease, chicken salmonellosis, influenza, and so forth.

In order to prevent and treat these infectious diseases, and for the purpose of gaining body weight and growth promotion of livestock animals, antibiotics have hitherto been used, but the public health problem concerning remains of drugs in meat etc., and the problem of appearance of multi-drug resistant strains have been pointed out. Furthermore, it is known that long term administration of an antibiotic forms a unique bacterial flora in which bacteria resistant to the antibiotic preferentially increase, and such a flora causes new diseases. In light of these circumstances, use of antibiotics as growth-promoting agents was blanket-banned from January, 2006 in Europe. Other countries also tend to ban or restrict use of antibiotics, and safe drugs that can replace antibiotics are desired.

One class of those developed as substitution of such antibiotics consists of live cell preparations (probiotics). Live cell preparations are for delivering live safe and useful bacteria such as lactic acid bacteria to intestines of livestock animals etc. by administering them together with feed, making them compete with harmful bacteria through production of metabolites such as organic acids in the intestines to eliminate the bacteria, thereby improving intestinal bacterial flora and preventing diseases (for example, Patent document 1).

It is also known for many years that, for example, the Freund's complete adjuvant comprising dead tubercle bacillus cells suspended in mineral oil has an immunostimulating action. Furthermore, there are also known drugs for suppressing infection of pathological bacteria utilizing bacterial cells sterilized by heating or the like. These mainly utilize the actions of cell wall components (peptidoglycans, teichoic acid, lipopolysaccharides, etc.) of the bacterial cells as active ingredients for activating host immune function, and they also have an advantage that the safety of the bacterial cells themselves is enhanced by heating. However, these live cell preparations and dead cell preparations have drawbacks of the mild effect thereof, which requires a large dose, high production cost thereof, which results in high product price, and so forth. Therefore, an alternative agent for antibiotics showing higher activity is desired.

By the way, concerning such an immunostimulant utilizing bacterial cells sterilized by heating or the like as described above, there is known, for example, a method for producing a lactic acid bacteria-containing composition for immunostimulation having an IL-12 production inducing activity, which comprises culturing lactic acid bacteria belonging to the genus *Lactobacillus,* immediately sterilizing the bacteria when decrease in pH of culture medium substantially ceased, and adding the obtained dead cells to a food or drink (for example, Patent document 2). In this method, the lactic acid bacteria are sterilized by heating when decrease in pH of culture medium substantially ceased, for example, within 30 minutes from the time point that pH decreases to a level slightly lower than 4. Sterilization of stored lactic acid bacteria of which growth is rested before the storage does not meet the definition of "immediately" of the aforementioned method.

Furthermore, there has also been reported that a sterilization or cell disruption product of bacteria belonging to the genus *Bacillus, Brevibacterium, Corynebacterium, Escherichia, Lactobacillus, Streptococcus, Streptomyces,* or *Bifidobacterium* has a prophylactic or therapeutic effect for virus diseases of fishes and crustaceans (Patent document 3).

However, it is not known that sterilization of coryneform bacteria or enterobacteria by heating improves immunostimulating action of the dead bacterial cells.

### Prior art References

### Patent documents

Patent document 1: Japanese Patent Laid-open (KOKAI) No. 10-167972
Patent document 2; Japanese Patent Laid-open No. 2010-95465
Patent document 3: Japanese Patent Laid-open No. 6-181656

### Summary of the Invention

### Object to be Achieved by the Invention

An object of the present invention is to provide an inexpensive, safe and highly active immunostimulant that can prevent or ameliorate infectious diseases of mammals, fowls, fishes, crustaceans and so forth by enhancing immunity of these animals without using antibiotics.

### Means for Achieving the Object

The inventors of the present invention conducted various researches, as a result, found that the immunostimulating effect of cells of coryneform bacteria or enterobacteria could be markedly enhanced by a heat treatment under an acidic condition, and precisely adjusted the conditions therefor to accomplish the present invention.

The present invention thus provides the followings.
(1) An immunostimulant for a mammal, fowl, fish, or crustacean, which comprises sterilized bacterial cells obtained by heat treating cells of a coryneform bacterium or an enterobacterium under a condition of pH 2 to 5.
(2) The immunostimulant as mentioned above, wherein pH is 3 to 5.
(3) The immunostimulant as mentioned above, wherein the heat treating is performed at 90 to 120°C for 3 minutes to 4 hours.
(4) The immunostimulant as mentioned above, wherein the cells of the coryneform bacterium or enterobacterium are derived from a fermentation by-product containing the bacterium.
(5) The immunostimulant as mentioned above, wherein the coryneform bacterium is *Corynebacterium glutamicum.*
(6) The immunostimulant as mentioned above, wherein the enterobacterium is *Escherichia coli* or *Pantoea ananatis.*
(7) The immunostimulant as mentioned above, wherein the coryneform bacterium or the enterobacterium is an L-amino acid or nucleic acid-producing bacterium.
(8) A feed for a mammal, fowl, fish or crustacean, which contains the immunostimulant as mentioned above.
(9) The feed as mentioned above, which contains 0.01 to 10% by weight of the immunostimulant as mentioned above.
(10) A method for producing an immunostimulant for a mammal, fowl, fish, or crustacean, which comprises heat treating cells of a coryneform bacterium or an enterobacterium under a condition of pH 2 to 5, and using the obtained sterilized cells as an active ingredient.
(11) A method for immunostimulation, which comprises administering the immunostimulant or feed as mentioned above to a mammal, fowl, fish, or crustacean.
(12) The method as mentioned above, wherein infection of a bacterium or a virus to the mammal, fowl, fish, or crustacean is prevented by activation of immunity, and the bacterium or virus is selected from the group consisting of verotoxin-producing *Escherichia coli, Salmonella* bacterium, *Mycoplasma* bacterium, *Clostridium* bacterium, *Lawsonia* bacterium, *Streptococcus* bacterium, new type *Streptococcus* bacterium, *Vibrio* bacterium, *Flexibacter maritimus,* white spot syndrome virus, and yellowhead virus.

### Embodiments for Carrying out the Invention

Hereafter, the present invention will be explained in detail.

The immunostimulant of the present invention comprises sterilized cells obtained by heat treating cells of a coryneform bacterium or an enterobacterium under a condition of pH 2 to 5.

The coryneform bacteria are aerobic, high GC content gram-positive bacilli. The coryneform bacteria include bacteria which have previously been classified into the genus *Brevibacterium* but are presently united into the genus *Corynebacterium* (Int. J. Syst. Bacteriol. 41:255 (1991)), and bacteria belonging to the genus *Brevibacterium,* which are closely related to the genus *Corynebacterium.*

Examples of the coryneform bacteria include the followings:
*Corynebacterium acetoacidophilum*
*Corynebacterium acetoglutamicum*
*Corynebacterium alkanolyticum*
*Corynebacterium callunae*
*Corynebacterium glutamicum (Brevibacterium lactofermentum)*
*Corynebacterium lilium*
*Corynebacterium melassecola*
*Corynebacterium thermoaminogenes*
*Corynebacterium herculis*
*Brevibacterium divaricatum*
*Brevibacterium flavum*
*Brevibacterium immariophilum*
*Brevibacterium lactofermentum*
*Brevibacterium roseum*
*Brevibacterium saccharolyticum*
*Brevibacterium thiogenitalis*
*Corynebacterium ammoniagenes*
*Brevibacterium album*
*Brevibacterium cerinum*
*Microbacterium ammoniaphilum*

Specific examples of the bacteria include the following strains.
*Corynebacterium acetoacidophilum* ATCC 13870
*Corynebacterium acetoglutamicum* ATCC 15806
*Corynebacterium alkanolyticum* ATCC 21511
*Corynebacterium callunae* ATCC 15991
*Corynebacterium glutamicum* ATCC 13020, ATCC 13032, ATCC 13060, ATCC 13869, FERM BP-734
*Corynebacterium lilium* ATCC 15990
*Corynebacterium melassecola* ATCC 17965
*Corynebacterium efficiens* AJ12340 (FERM BP-1539)
*Corynebacterium herculis* ATCC 13868
*Brevibacterium divaricatum* ATCC 14020
*Brevibacterium flavum* ATCC 13826, ATCC 14067, AJ12418 (FERM BP-2205)
*Brevibacterium immariophilum* ATCC 14068
*Brevibacterium lactofermentum* ATCC 13869
*Brevibacterium roseum* ATCC 13825
*Brevibacterium saccharolyticum* ATCC 14066
*Brevibacterium thiogenitalis* ATCC 19240
*Brevibacterium ammoniagenes* ATCC 6871, ATCC 6872
*Brevibacterium album* ATCC 15111
*Brevibacterium cerinum* ATCC 15112
*Microbacterium ammoniaphilum* ATCC 15354

The enterobacterium is not particularly limited so long as the bacterium belongs to the family *Enterobacteriaceae* such as those of the genera *Escherichia, Enterobacter, Pantoea, Klebsiella, Serratia, Erwinia, Salmonella* and *Morganella.* Specifically, those classified into the family *Enterobacteriaceae* according to the taxonomy used in the NCBI (National Center for Biotechnology Information) database (http://www.ncbi.nlm.nih.gov/htbin-post/Taxonomy/wgetorg?mode=Tree&id=1236&lvl=3&keep=1&srchmod e=1&unlock) can be used. As the enterobacterium, it is desirable to use a bacterium of the genus *Escherichia.*

Although the *Escherichia* bacterium is not particularly limited, specifically, those described in the work of Neidhardt et al. (Backmann B.J., 1996, Derivations and Genotypes of some mutant derivatives of Escherichia coli K-12, p.2460-2488, Table 1, In F.D. Neidhardt (ed.), Escherichia coli and Salmonella Cellular and Molecular Biology/Second Edition, American Society for Microbiology Press, Washington, D.C.) can be used. Examples of *Escherichia* bacteria include, for example, *Escherichia coli.* Specific examples of *Escherichia coli* include those strains derived from the *Escherichia coli* K12 strain, such as the *Escherichia coli* MG1655 strain (ATCC 47076), and the W3110 strain (ATCC 27325).

The strains attached with ATCC numbers are available from the American Type Culture Collection (Address: P.O. Box 1549, Manassas, VA 20108, United States of America). That is, registration numbers are given to each of the strains, and the strains can be ordered by using these registration numbers (refer to http://www.atcc.org/). The registration numbers of the strains are listed in the catalogue of the American Type Culture Collection.

Examples of the *Enterobacter* bacteria include *Enterobacter agglomerans, Enterobacter aerogenes* and so forth, and examples of the *Pantoea* bacteria include *Pantoea ananatis.* Some strains of *Enterobacter agglomerans* were recently reclassified into *Pantoea agglomerans, Pantoea ananatis,* or *Pantoea stewartii* on the basis of nucleotide sequence analysis of 16S rRNA etc. In the present invention, a bacterium belonging to any of the genus *Enterobacter* or *Pantoea* may be used so long as it is a bacterium classified into the family *Enterobacteriaceae.* When *Pantoea ananatis* is bred by using genetic engineering techniques, the *Pantoea ananatis* AJ13355 strain (FERM BP-6614), AJ13356 strain (FERM BP-6615), AJ13601 strain (FERM BP-7207), and derivatives thereof can be used. These strains were identified and deposited as *Enterobacter agglomerans* when they were isolated, but as described above, these strains have been reclassified as *Pantoea ananatis* on the basis of analysis of the nucleotide sequence of 16S rRNA and so forth.

Although the method for preparing cells of the coryneform bacterium or enterobacterium is not particularly limited, cells can be obtained by culturing a coryneform bacterium or enterobacterium in a medium in which these bacteria can grow under conditions under which these bacteria can grow.

As the medium, conventional media containing a carbon source, nitrogen source and mineral salts as well as organic trace nutrients such as amino acids and vitamins as required can be used. Either a synthetic medium or a natural medium may be used. Any kinds of carbon source and nitrogen source may be used for the medium, so long as the strain to be cultured can utilize them.

As the carbon source, for example, sugars such as glucose, glycerol, fructose, sucrose, maltose, mannose, galactose, starch hydrolysates and molasses can be used. In addition, organic acids such as acetic acid and citric acid, and alcohols such as ethanol can also be used each alone or in combination with other carbon sources.

As the nitrogen source, ammonia, ammonium salts such as ammonium sulfate, ammonium carbonate, ammonium chloride, ammonium phosphate and ammonium acetate, nitric acid salts and so forth can be used.

As the mineral salts, phosphoric acid salts, magnesium salts, calcium salts, iron salts, manganese salts and so forth can be used.

As the organic trace nutrients, amino acids, vitamins, fatty acids, nucleic acids, those containing the foregoing substances such as peptone, casamino acid, yeast extract, soybean protein decomposition product and so forth can be used. When an auxotrophic mutant strain that requires an amino acid or the like for its growth is used, it is preferable to supplement the required nutrient.

The culture is performed, for example, as aerobic culture, while the temperature is controlled to be 20 to 45°C, and pH to be 3 to 9. When pH decreases during the culture, for example, the medium is neutralized by adding calcium carbonate, or with an alkali such as ammonia gas. A sufficient amount of bacterial cells are obtained by culture under such conditions as described above preferably for about 10 to 120 hours.

Furthermore, as the bacterial cells, besides the cells obtained by the aforementioned preparation method, a fermentation by-product containing cells obtained in fermentative production of a target substance using a coryneform bacterium or enterobacterium can also be used. The fermentation by-product is not particularly limited so long as it contains the bacterial cells, and examples include a medium remained after a target substance is collected, solid content obtained from the medium by centrifugation or filtration, and fractionation products thereof containing the cells. Furthermore, the fermentation by-product may be obtained after the culture is completed, or may be obtained from the medium continuously or intermittently drawn from a fermentation tank during the fermentation.

Examples of the target substance include, for example, amino acids, nucleic acids, and so forth.

Examples of the amino acids include L-lysine, L-ornithine, L-arginine, L-histidine, L-citrulline, L-isoleucine, L-alanine, L-valine, L-leucine, L-glycine, L-threonine, L-serine, L-proline, L-phenylalanine, L-tyrosine, L-tryptophan, L-cysteine, L-cystine, L-methionine, L-glutamic acid, L-asparatic acid, L-glutamine, and L-asparagine.

Examples of the nucleic acid include purine nucleosides, purine nucleotides, and so forth. The purine nucleosides include inosine, xanthosine, guanosine, adenosine, and so forth, and the purine nucleotides include 5'-phosphate esters of the purine nucleosides, for example, inosinic acid, (inosine-5'-phosphate, henceforth also referred to as "IMP"), xanthylic acid (xanthosine-5'-phosphate, henceforth also referred to as "XMP"), guanylic acid (guanosine-5'-monophosphate, henceforth also referred to as "GMP"), adenylic acid (adenosine-5'-monophosphate, henceforth also referred to as "AMP"), and so forth.

As the coryneform bacterium or enterobacterium used for the present invention, a coryneform bacterium or enterobacterium bred by the following methods can be used.

In order to impart L-amino acid-producing ability, methods conventionally employed in the breeding of amino acid-producing strains of coryneform bacteria or *Escherichia* bacteria (see "Amino Acid Fermentation", Gakkai Shuppan Center (Ltd.), 1st Edition, published May 30, 1986, pp. 77-100) can be used. Such methods include methods of acquiring auxotrophic mutants, strains resistant to L-amino acid, or metabolic regulation mutants, or constructing a recombinant strain so that it overexpresses an L-amino acid biosynthesis enzyme. In the breeding of an L-amino acid-producing bacteria, one or more of the above described properties such as auxotrophy, analogue-resistance, or metabolic regulation mutation may be imparted. Expression of one or more L-amino acid biosynthesis enzymes may be enhanced. Furthermore, impartation of such properties as auxotrophy, analogue resistance, or metabolic regulation mutation may be combined with enhancement of a biosynthesis enzyme.

An auxotrophic mutant strain, L-amino acid analogue-resistant strain, or metabolic regulation mutant strain having an ability to produce an L-amino acid can be obtained by subjecting a parent strain or wild-type strain to a conventional mutagenesis, such as exposure to X-rays or UV irradiation, or treatment with a mutagen such as N-methyl-N'-nitro-N-nitrosoguanidine, etc., and then selecting those which exhibit an autotrophy, analogue resistance, or metabolic regulation mutation, and which also have the ability to produce an L-amino acid from the obtained mutant stains.

Moreover, the L-amino acid-producing ability can also be imparted or enhanced by increasing an enzymatic activity by gene recombination. An example of the method for increasing enzymatic activity includes modifying the bacterium so that the expression of a gene coding for an enzyme involved in the biosynthesis of an L-amino acid is enhanced. Gene expression can also be increased by introducing an amplification plasmid prepared by introducing a DNA fragment containing the gene into an appropriate plasmid which contains, for example, at least a gene responsible for replication and proliferation of the plasmid in the microorganism, increasing the copy number of the gene on the chromosome by conjugation, transfer, or the like, or introducing a mutation into the promoter region of the gene (refer to International Publication WO95/34672).

When an objective gene is introduced into the aforementioned amplification plasmid or chromosome, any promoter can be used to express the gene so long as the chosen promoter functions in coryneform bacteria. The promoter can be the native promoter for the gene, or a modified promoter. Expression of a gene can also be controlled by suitably choosing a promoter that strongly functions in coryneform bacteria, or by making the -35 and - 10 regions of the promoter closer to the consensus sequence. Such methods as described above for enhancing expression of enzyme genes are described in International Publication WO00/18935, European Patent Publication No. 1010755, and so forth.

### L-Threonine-producing bacteria

Examples of L-threonine-producing bacteria include, but are not limited to, strains belonging to the genus *Escherichia,* such as *E. coli* TDH-6/pVIC40 (VKPM B-3996) (U.S. Patent No. 5,175,107, U.S. Patent No. 5,705,371), *E. coli* 472T23/pYN7 (ATCC 98081) (U.S. Patent No. 5,631,157), *E*. *coli* NRRL-21593 (U.S. Patent No. 5,939,307), *E. coli* FERM BP-3756 (U.S. Patent No. 5,474,918), *E. coli* FERM BP-3519 and FERM BP-3520 (U.S. Patent No. 5,376,538), *E. coli* MG442 (Gusyatiner et al., Genetika (in Russian), 14, 947-956 (1978)), *E. coli* VL643 and VL2055 (EP 1149911 A), *E. coli* VKPM B-5318 (EP 0593792 B), and so forth.

### L-Lysine-producing bacteria

Specific examples of L-lysine-producing bacteria include the *Escherichia coli* AJ11442 strain (FERM BP-1543, NRRL B-12185, see Japanese Patent Laid-open No. 56-18596 and U.S. Patent No. 4,346,170), *Escherichia coli* VL611 strain (Japanese Patent Laid-open No. 2000-189180), and so forth. As an L-lysine-producing *Escherichia coli,* the WC196 strain (see International Publication WO96/17930), and the *Escherichia coli* WC196ΔcadAΔldcC/pCABD2 strain can also be used.

Examples of L-lysine producing coryneform bacteria include AEC-resistant mutant strains (*Brevibacterium lactofermentum* AJ11082 (NRRL B-11470) strain etc., refer to Japanese Patent Publication (KOKOKU) Nos. 56-1914, 56-1915, 57-14157, 57-14158, 57-30474, 58-10075, 59-4993, 61-35840, 62-24074, 62-36673, 5-11958, 7-112437 and 7-112438); mutant strains requiring an amino acid such as L-homoserine for their growth (refer to Japanese Patent Publication Nos. 48-28078 and 56-6499); mutant strains showing resistance to AEC and further requiring an amino acid such as L-leucine, L-homoserine, L-proline, L-serine, L-arginine, L-alanine and L-valine (refer to U.S. Patent Nos. 3,708,395 and 3,825,472); L-lysine-producing mutant strains showing resistance to DL-α-amino-ε-caprolactam, α-amino-lauryllactam, aspartic acid analogue, sulfa drug, quinoid or N-lauroylleucine; L-lysine-producing mutant strains showing resistance to oxaloacetate decarboxylase or a respiratory tract enzyme inhibitor (Japanese Patent Laid-open Nos. 50-53588, 50-31093, 52-102498, 53-9394, 53-86089, 55-9783, 55-9759, 56-32995, 56-39778, Japanese Patent Publication Nos. 53-43591 and 53-1833); L-lysine-producing mutant strains requiring inositol or acetic acid (Japanese Patent Laid-open Nos. 55-9784 and 56-8692); L-lysine-producing mutant strains that are susceptible to fluoropyruvic acid or a temperature of 34°C or higher (Japanese Patent Laid-open Nos. 55-9783 and 53-86090); L-lysine-producing mutant strains of *Brevibacterium* or *Corynebacterium* bacteria showing resistance to ethylene glycol (U.S. Patent No. 4,411,997), and so forth.

### L-Glutamic acid-producing bacteria

Examples of L-glutamic acid-producing bacteria and parent strains which can be used to derive L-glutamic acid-producing bacteria include *Escherichia* bacterial strains such as *E. coli* VL334thrC⁺ (EP 1172433).
*E. coli* W3110sucA::Km^{r}
*E. coli* AJ12624 (FERM BP-3853)
*E. coli* AJ12628 (FERM BP-3854)
*E. coli* AJ12949 (FERM BP-4881)
*E. coli* AJ13199 (FERM BP-5807) (U.S. Patent No. 5,908,768)
FFRM P-12379 (U.S. Patent No. 5,393,671)
AJ13138 (FERM BP-5565) (U.S. Patent No. 6,110,714), and so forth.

Examples of coryneform bacteria having L-glutamic acid-producing ability include, for example, the following strains:
*Brevibacterium lactofermentum* L30-2 strain (Japanese Patent Laid-open No. 2006-340603)
*Brevibacterium lactofermentum* ΔS strain (WO95/34672)
*Brevibacterium lactofermentum* AJ12821 (FERM BP-4172, French Patent No. 9401748)
*Brevibacterium flavum* AJ12822 (FERM BP-4173, French Patent No. 9401748)
*Corynebacterium glutamicum* AJ12823 (FERM BP-4174, French Patent No. 9401748)
*Corynebacterium glutamicum* L30-2 strain (Japanese Patent Laid-open No. 2006-340603)
*Brevibacterium flavum* AJ3949 (FERM BP-2632, refer to Japanese Patent Laid-open No. 50-113209)
*Corynebacterium glutamicum* AJ11628 (FERM P-5736, refer to Japanese Patent Laid-open No. 57-065198)
*Brevibacterium flavum* AJ11355 (FERM P-5007, refer to Japanese Patent Laid-open No. 56-1889)
*Corynebacterium glutamicum* AJ11368 (FERM P-5020, refer to Japanese Patent Laid-open No. 56-1889)
*Brevibacterium flavum* AJ11217 (FERM P-4318, refer to Japanese Patent Laid-open No. 57-2869)
*Corynebacterium glutamicum* AJ11218 (FERM P-4319, refer to Japanese Patent Laid-open No. 57-2869)
*Brevibacterium flavum* AJ11564 (FERM P-5472, refer to Japanese Patent Laid-open No. 56-140895)
*Brevibacterium flavum* AJ11439 (FERM P-5136, refer to Japanese Patent Laid-open No. 56-35981)
*Corynebacterium glutamicum* H7684 (FERM BP-3004, refer to Japanese Patent Laid-open No. 04-88994)
*Brevibacterium lactofermentum* AJ11426 (FERM P-5123, refer to Japanese Patent Laid-open No. 56-048890)
*Corynebacterium glutamicum* AJ11440 (FERM P-5137, refer to Japanese Patent Laid-open No. 56-048890)
*Brevibacterium lactofermentum* AJ11796 (FERM P-6402, refer to Japanese Patent Laid-open No. 58-158192)

Examples of L-glutamic acid-producing bacterium of *Pantoea ananatis* include the *Pantoea ananatis* AJ13355 strain. This strain was isolated from soil in Iwata-shi, Shizuokaken, Japan, and was identified as being able to proliferate in a medium containing L-glutamic acid and a carbon source at a low pH. The *Pantoea ananatis* AJ13355 strain was deposited at the independent administrative agency, National Institute of Advanced Industrial Science and Technology, International Patent Organism Depositary (Tsukuba Central 6, 1-1, Higashi 1-Chome, Tsukuba-shi, Ibaraki-ken, 305-8566, Japan) on February 19, 1998 and assigned an accession number of FERM P-16644. It was then converted to an international deposit under the provisions of Budapest Treaty on January 11, 1999 and assigned an accession number of FERM BP-6614. This strain was originally identified as *Enterobacter agglomerans* when it was isolated, and deposited as *Enterobacter agglomerans* AJ13355. However, it was recently re-classified as *Pantoea ananatis* on the basis of nucleotide sequencing of 16S rRNA and so forth.

Furthermore, examples of L-glutamic acid-producing bacterium of *Pantoea ananatis* also include the AJ13356 strain (U.S. Patent No. 6,331,419), and the SC17sucA strain (U.S. Patent No. 6,596,517). The AJ13356 strain was deposited at the National Institute of Bioscience and Human-Technology, Agency of Industrial Science and Technology (currently, the independent administrative agency, National Institute of Advanced Industrial Science and Technology, International Patent Organism Depositary (Tsukuba Central 6, 1-1, Higashi 1-Chome, Tsukuba-shi, Ibaraki-ken, 305-8566, Japan) on February 19, 1998, and assigned an accession number of FERM P-16645. Then, the deposit was converted into an international deposit under the provisions of the Budapest Treaty on January 11, 1999, and assigned an accession number of FERM BP-6616. Although the AJ13355 and AJ13356 strains were deposited at the aforementioned depository as *Enterobacter agglomerans,* they are referred to as *Pantoea ananatis* in this specification. The SC17sucA strain was assigned a private number of AJ417, and deposited at the National Institute of Advanced Industrial Science and Technology, International Patent Organism Depositary on February 26, 2004, under an accession number of FERM BP-08646.

Examples of L-glutamic acid-producing bacterium of *Pantoea ananatis* further include the SC17sucA/RSFCPG+pSTVCB strain (WO0200901693), AJ13601 strain (accession number FERM BP-7207), NP106 strain (WO0200901693), and NA1 strain (WO0200901693).

### Nucleic acid-producing bacteria

Examples of 5'-inosinic acid-producing bacteria include *Corynebacterium ammoniagenes* ATCC 6872, and *Corynebacterium ammoniagenes* KCCM10226 (Japanese Patent No. 4173368). Examples of 5'-inosine-producing bacteria include *Corynebacterium ammoniagenes* KCCM10905 (WO2009/088184). Examples of xanthylic acid-producing bacteria include *Corynebacterium ammoniagenes* CJXFT0301 (deposition number: KCCM10530, Japanese Patent No. 4447609), and *Corynebacterium ammoniagenes* CJXSP 0201 KCCM10448 (WO2004/053110).

As the bacterial cells of coryneform bacterium or enterobacterium of the present invention, cells obtained by culturing a bacterium bred as described above may be used, and commercially available dry cells by-produced in amino acid or nucleic acid fermentation, for example, Ajitein (Indonesia Ajinomoto Co., Inc. and Thailand Ajinomoto Co., Inc.), Protorsan (Europe Ajinomoto Co., Inc.), etc., which are sold as single cell proteins, may also be used.

The cells of coryneform bacterium or enterobacterium obtained as described above are subjected to a heat treatment under an acid condition. The cells to be heat treated may be dry cells or wet cells, but they are suspended in an aqueous solvent prior to the heat treatment. Examples of the aqueous solvent include water, buffer, and so forth. Although the cell density in the suspension is not particularly limited, it is, for example, 0.1 to 40% by weight.

The pH of the cell suspension is adjusted to be an acidic pH, preferably weakly acidic pH, for example, pH 2 to 5, preferably pH 2 to 4, or pH 3 to 5, particularly preferably 3 to 4. The acid used for adjusting pH is not particularly limited, and examples include inorganic acids such as sulfuric acid, nitric acid and hydrochloric acid, and organic acids such as acetic acid.

The conditions of the heat treatment may be, for example, 90 to 120°C, preferably 95 to 115°C, more preferably 100 to 110°C, and heating time of 3 minutes to 4 hours, preferably 30 minutes to 3 hours, more preferably 1 to 3 hours. More specifically, the heat treatment may be performed, for example, at 105°C for 2 hours. The heating method is not particularly limited, and it may be performed by, for example, autoclaving.

After the heat treatment, the cells are collected from the cell suspension as a pellet by filtration, centrifugation, or the like, concentrated, and dried as required to give sterilized cells. The cells may be collected after pH of the cell suspension is adjusted to, for example, a neutral pH.

The sterilized cells obtained as described above can be used as an immunostimulant as they are, or after being mixed with an arbitrary carrier. The cells contained in the immunostimulant may contain cells of one or two or more kinds of bacteria.

Immunity of a mammal, fowl, fish, or crustacean can be enhanced by administering the immunostimulant or feed of the present invention to such an animal as described above. Immunostimulation means enhancement of immune function of an animal, and by immunostimulation, infectious diseases caused by infection of bacteria or viruses can be prevented or treated, or prophylactic or therapeutic effect can be enhanced. The animal includes mammals, fowls, fishes, and crustaceans.

The immunostimulating activity can be evaluated by observing, for example, antibody production by antibody-producing cells, production of cytokine such as IL-12 by immune system cells, phagocytic ability of phagocytes such as macrophages and neutrophiles, or the like, as an index.

Form of the immunostimulant is not particularly limited, and it may be in the form of any of liquid, paste, powder, and solid.

Furthermore, the carrier is not also particularly limited, so long as a carrier that can be orally ingested by mammals, fowls, fishes, crustaceans and so forth is used.

The immunostimulant of the present invention is orally administered directly or by adding it to feed. The immunostimulant can be easily administered to a mammal, fowl, fish, crustacean or the like by mixing it with feed for these animals.

Examples of the mammal include livestock animals such as ox or cow, pig, horse, sheep, and goat, pet animals such as dog and cat, examples of fowls include domestic fowls such as chicken, quail, duck, goose, and turkey, and pet fowls such as parakeet, parrot, paddybird and canary, examples of fishes include farmed fishes such as tuna, bonito, yellowtail, greater amberjack (*Seriola dumerili*)*,* yellowtail amberjack (*Seriola lalandi*)*,* bream, salmon, cod, trout, rainbow trout, bastard halibut (*Paralichthys olivaceus*), tiger globefish, filefish, horse mackerel, grouper, tilapia, catfish, eel, carp, grass carp, silver carp, and crucian carp, and examples of crustaceans include Japanese tiger prawn, black tiger prawn, vannamei shrimp, crab, and so forth.

Examples of pathogenic microbe of infectious diseases which may be prevented or treated by immunostimulation include verotoxin-producing *Escherichia coli, Salmonella* bacteria, *Mycoplasma* bacteria, *Clostridium* bacteria, *Lawsonia* bacteria, and so forth concerning stockbreeding and pet breeding, as well as *Streptococcus* bacteria (*Streptococcus pyogenes* etc.), new type *Streptococcus* bacterium (*Streptococcus dysgalactiae*)*, Vibrio* bacteria *(Vibrio anguillarum, Vibrio ordalii), Flexibacter maritimus,* white spot syndrome virus of Japanese tiger prawn, yellowhead virus, and so forth concerning fishery.

Form of the feed is not particularly limited. A feed material usually used for feeding the aforementioned animals such as liquid feed, powder feed, solid feed, moist pellet, dry pellet, extruder pellet, and live bait can be appropriately chosen and prepared depending on the objective animal, and the immunostimulant of the present invention can be mixed with it. For solid feed such as pellet, the immunostimulant of the present invention can be efficiently added by coating it with a tackifier such as guar gum.

As the feed material, fish meal, bone meal, skim milk, cottonseed meal, wheat flour, wheat germ, rice bran, brewer's yeast, vitamins, soybean meal, plant residue, and so forth are generally used. The aforementioned immunostimulant is added to feed at a ratio of, for example, 0.001 to 10%, preferably 0.005 to 10%, more preferably 0.01 to 10%, in terms of a ratio of dry weight of the sterilized bacterial cells based on the dry weight of the feed.

In particular, for use in culture fishery, the ratio is, for example, 0.001 to 10%, preferably 0.005 to 10%, more preferably 0.005 to 5%.

For use in pig raising, the ratio is, for example, 0.01 to 10%, preferably 0.01 to 1%, more preferably 0.1 to 0.2%, and for used in chicken raising, the ratio is, for example, 0.001 to 10%, preferably 0.01 to 1%, more preferably 0.05 to 0.2%.

As for feed for fishery, the immunostimulant may be indirectly administered by administering it to live bait. For example, it can be administered to live bait such as *Artemia* sp. or *Phylum rotifera,* and fishes or crustaceans can be allowed to bait the live bait to enhance the immunostimulating effect.

Administration time of the immunostimulant or feed is not particularly limited, and it may be administered before or after infection with a pathogenic microbe. However, it is preferably administered before infection of a pathogenic microbe is expected. For example, in the case of using coryneform bacteria, daily dose is 0.1 to 1000 mg, preferably 1 to 100 mg, per 1 kg of body weight of pig, fowl, shrimp or fish. For use in culture fishery feed, daily dose is preferably 30 to 100 mg, and for use in stockbreeding, daily dose is preferably 40 to 120 mg, but the dose is not limited to be within such ranges.

In particular, in the case of using *Escherichia coli,* the daily dose is 10 to 50 mg, preferably 15 to 30 mg, per 1 kg of body weight of pig, fowl, shrimp or fish.

### Examples

Hereinafter, the present invention will be specifically explained with reference to examples.

### Example 1: Investigation of influence of pH for heat treatment of bacterial cells

Distilled water was added to dry cells of *Corynebacterium glutamicum* by-produced in fermentative production of L-glutamic acid using the bacterium, so that the mixture contained 10% by weight of the cells, and the cells were sufficiently suspended. The abovementioned dry cells had been obtained by collecting cells from fermentation broth, and drying them on a drum under conditions of about pH 6 and 60 to 70°C.

Then, the cell suspension was adjusted to pH 2, 3, 4, 5, 6, 7, or 8 using sulfuric acid or sodium hydroxide, and heated at 105°C for 2 hours by using an electric autoclave to prepare cell samples heat-treated at the various pH values. Immunostimulation of these samples was investigated by the in vitro methods described below as test methods 1 and 2.

### (Test method 1) Evaluation of immunostimulating activity using IgA production in mouse small intestine Peyer's patch cells as index

### 1-1. Extraction of Peyer's patches and preparation of cell suspension

A mouse was dissected, and the small intestine was extracted. Peyer's patches were extracted from the extracted small intestine by using a curved ophthalmic scissors or the like. A single cell suspension was prepared from the Peyer's patches by using a cell strainer (produced by Becton, Dickinson and Company), and the single cells were collected by centrifugation, and suspend in the RPMI 1640 medium containing 10% of fetal calf serum.

### 1-2. Cell culture

The number of live cells in the cell suspension was measured by using trypan blue staining. The cell count was adjusted to 1 x 10⁵ to 1 x 10⁶ cells/ml, and the suspension was poured into wells of a 96-well cell culture microplate in a volume of 200 µL per well. A heat-treated cell sample solution of which concentration was adjusted to 2000 µg/ml or 80 µg/ml with a PBS buffer, or a PBS buffer as a negative control was added in a volume of 5 µL to each well, and the cells were cultured at 37°C for 5 days under 5% CO₂. As a positive control, LPS (lipopolysaccharides derived from *E*. *coli,* Sigma) suspend in PBS at 5 µg/ml was used.

### 1-3. Measurement of IgA concentration

After completion of the culture, total IgA concentration in the culture supernatant was measured by using an ELISA kit (Bethyl). The measurement was carried out in duplicate, and average was calculated.

### (Test method 2) IL-12 production by mouse peritoneal macrophage

### 2-1. Extraction of peritoneal macrophages and preparation of cell suspension

An aqueous solution of glycerol at a concentration of 1 g/100 ml was intraperitoneally injected into mice in a volume of 0.4 ml/mouse, and the mice were bred overnight. Then, the mice were euthanized by cervical vertebra dislocation, cooled PBS was intraperitoneally injected in a volume of 5 ml/mouse, the abdominal part was sufficiently massaged, and then the intra-abdominal solution (about 4 ml) was extracted with a syringe, and centrifuged in a silicon-coated spitz tube (1200 rpm, 5 minutes). After the supernatant and the erythrocytes on the wall surface of the spitz tube were removed, cold PBS was added, pipetting was performed, the suspension was centrifuged (800 rpm, 5 minutes), and the supernatant and the erythrocytes on the wall surface were removed. This washing operation was repeated twice. After the washing, the cells including macrophages were suspended in the RPMI 1640 medium containing 10% FCS.

### 2-2. Cell culture

The number of live cells in the cell suspension was measured by using trypan blue staining. The cell count was adjusted to 2 x 10⁶ cells/ml, and the suspension was poured into wells of a 96-well cell culture microplate in a volume of 100 µl per well. A heat-treated cell sample solution of which concentration was adjusted to 2000 µg/ml or 80 µg/ml with the RPMI 1640 medium containing 10% FCS was added in a volume of 100 µl to each well, and the cells were cultured at 37°C for 7 days under 5% CO₂. As a positive control, LPS (lipopolysaccharides derived from *E. coli,* Sigma) suspend at 5 µg/ml in the RPMI 1640 medium containing 10% FCS was used.

### 2-3. Measurement of IL-12p70 concentration

After completion of the culture, IL-12 concentration in the culture supernatant was measured by using an ELISA kit. The measurement was carried out in duplicate, and average was calculated.

The results obtained by these test methods are shown in Tables 1 and 2, respectively.

According to both the test methods 1 and 2, immunostimulating activity was enhanced under acidic conditions. Although enhancement of the immunostimulating activity was also observed at pH 2 and pH 6, it was especially remarkable at pH 3 to 5.

**Table 1**

| | pH for heat treatment | Bacterial cell amount (mg/ml) | |
|---|---|---|---|
| | | 1 | 0.04 |
| Test group | pH 2 | 0.82 | 0.44 |
| | pH 3 | 2.32 | 0.81 |
| | pH 4 | 2.20 | 1.27 |
| | pH 5 | 2.03 | 0.90 |
| | pH 6 | 1.92 | 0.75 |
| | pH 7 | 1.21 | 0.57 |
| Control group | Without heat treatment | 0.74 | 0.42 |

| | | | |
|---|---|---|---|
| (The numerals shown in the table indicate the total IgA concentrations (µg/ml) in the culture supernatants) | | | |

**Table 2**

| | pH for heat treatment | Bacterial cell amount (mg/ml) | |
|---|---|---|---|
| | | 1 | 0.04 |
| Test group | pH 2 | 11.8 | 15.0 |
| | pH 3 | 102.8 | 10.6 |
| | pH 4 | 63.9 | 15.4 |
| | pH 5 | 57.4 | 6.6 |
| | pH 6 | 35.4 | 6.6 |
| | pH 7 | 31.7 | 6.2 |
| Control group | Without heat treatment | 9.3 | 6.6 |

| | | | |
|---|---|---|---|
| (The numerals shown in the table indicate the total IL-12p70 concentrations (pg/ml) in the culture supernatants) | | | |

### Example 2: Investigation of influence of temperature for heat treatment of bacterial cells

In the same manner as that of Example 1, distilled water was added to the by-product dry cells, so that the mixture contained 10% by weight of the cells, and the cells were sufficiently suspended. The cell suspension was adjusted to pH 4 with sulfuric acid, and heated at three levels of temperature, 90°C, 105°C and 120°C, for 2 hours by using an electric autoclave to prepare heat-treated cell samples treated at the various temperatures under acidic condition. Immunostimulating activity of these samples was investigated by the aforementioned test methods 1 and 2. The results are shown in Tables 3 and 4.

According to both the test methods 1 and 2, immunostimulating activity was enhanced at all the temperatures, but the enhancement was especially remarkable at 105°C.

**Table 3**

| | Temperature for heat treatment | Bacterial cell amount (mg/ml) | |
|---|---|---|---|
| | | 1 | 0.04 |
| Test group | 90°C | 0.88 | 0.69 |
| | 105°C | 2.20 | 1.27 |
| | 120°C | 0.79 | 0.78 |
| Control group | Without heat treatment | 0.74 | 0.42 |

| | | | |
|---|---|---|---|
| (The numerals shown in the table indicate the total IgA concentrations (µg/ml) in the culture supernatants) | | | |

**Table 4**

| | Temperature for heat treatment | Bacterial cell amount (mg/ml) | |
|---|---|---|---|
| | | 1 | 0.04 |
| Test group | 90°C | 27.1 | 7.8 |
| | 105°C | 63.9 | 15.4 |
| | 120°C | 20.1 | 20.5 |
| Control group | Without heat treatment | 9.3 | 6.6 |

| | | | |
|---|---|---|---|
| (The numerals shown in the table indicate the total IL-12p70 concentrations (pg/ml) in the culture supernatants) | | | |

### Example 3: Pathogenic germ infection prevention effect of bacterial cells heat treated under acidic condition

Bacterial infection prevention effects of samples obtained by subjecting the bacterial cells as a fermentation by-product to a heat treatment (105°C, 2 hours) under a weakly acidic condition (pH 4) in the same manner as that of Example 1 (henceforth referred to as "bacterial cells heat-treated under acidic condition, test group I") and samples comprising the bacterial cells not subjected to any heat treatment (henceforth referred to as "non-treated bacterial cells, test group II") were examined in mice by the test method 3 described below. As control groups, a group of mice administered with physiological saline instead of the bacterial cells (negative control, control group I), a group of mice administered with 400 mg/kg of an antibiotic bicozamycin (Schering-Plough Animal Health; trade name, Bacteron) (positive control, control group II), and a no infection group (administered with physiological saline, control group III) were used.

### (Test method 3) Infection prevention effect in mouse Escherichia coli infection model

### 3-1. Breeding of mice

BALB/c female mice (21 days old, Japan SLC) as test animals were introduced into a room in which temperature was controlled to be 25°C, and then randomly divided into groups, and mice of each group were put into a mouse breeding cage, and bred by using MF produced by Oriental Yeast as a basal diet. The fourth day from the start of breeding (day of inoculation of bacteria to be infected) was defined as the test starting day, and from the breeding starting day (4 days before the test starting day) to the day 17 (13th day after the start of the test), predetermined amounts of test samples were each forcibly orally administered to mice of each group at 10:00 in the morning every day. Cyclophosphamide was intraperitoneally administered in an amount of 200 mg/kg, 2 days before the start of the test and on the test starting day.

### 3-2. Administration of challenge bacterium

On the test starting day (day 0 after the start), 1.9 x 10⁶ cells/animal of a challenge bacterium (verotoxin-producing *Escherichia coli* derived from pig, Miyazaki University strain No. 1362-1) suspended in sterilized physiological saline were intraperitoneally administered to the mice 4 hours after the administration of cyclophosphamide. The bacterium was further intraperitoneally administered in an amount of 1.9 x 10⁶ cells/animal each on the day 1 and day 2 after the start of the test.

### 3-3. Determination of effect

The mice were observed every day during the test period, and if any mouse died, it was collected and recorded.

The results are shown in Table 5. In two of the groups, one mouse died before the inoculation of the pathogenic bacterium, and therefore the test was performed with 9 mice for these groups.

**Table 5**

| | Dose (/kg body weight/day) | Transition of living mice number from pathogenic bacterium inoculation day (day 0 after start of test) (number of mice) | | | | | | | |
|---|---|---|---|---|---|---|---|---|---|
| | | Day 0 | Day 2 | Day 3 | Day 4 | Day 5 | Day 7 | Day 10 | Day 13 |
| Test group I: Bacterial cells heat-treated under acidic condition | 1 mg | 10 | 10 | 6 | 2 | 2 | 2 | 2 | 2 |
| | 10 mg | 10 | 10 | 10 | 10 | 10 | 10 | 10 | 10 |
| | 100 mg | 10 | 10 | 7 | 6 | 6 | 6 | 6 | 6 |
| Test group II: Non-treated bacterial cells | 1 mg | 9* | 9 | 9 | 3 | 2 | 2 | 1 | 1 |
| | 10 mg | 10 | 10 | 6 | 0 | 0 | 0 | 0 | 0 |
| | 100 mg | 10 | 10 | 10 | 10 | 9 | 9 | 9 | 9 |
| Control group I (physiological saline) | - | 10 | 10 | 7 | 6 | 2 | 2 | 2 | 2 |
| Control group II (administered with Bacteron) | 400 mg | 9* | 9 | 9 | 9 | 9 | 9 | 9 | 9 |
| Control group III (no infection) | - | 10 | 10 | 10 | 10 | 10 | 10 | 10 | 10 |

| | | | | | | | | | |
|---|---|---|---|---|---|---|---|---|---|
| (* One mouse died before the inoculation of the pathogenic bacterium, and therefore the test was performed with 9 mice/group) | | | | | | | | | |

It was confirmed that the number of deaths of the mice due to verotoxin-producing *Escherichia coli* was decreased by administration of 10 mg/kg of body weight or more of the bacterial cells heat-treated under acidic condition or 100 mg/kg of the non-treated bacterial cells. In particular, it was observed that the bacterial cells heat-treated under acidic condition exhibited the effect with a lower dose as compared to the non-treated bacterial cells. In both the test groups I and II, clinical abnormality due to the bacterial cell administration was not observed.

### Example 4: Investigation of influence of pH for heat treatment of bacterial cells using washed cells

In order to eliminate influences of the components of the culture medium and the products secreted in the medium during the heat treatment of the bacterial cells, washed bacterial cells of the *Corynebacterium glutamicum* AJ1511 strain (ATCC 13869) were used instead of the by-product dry cells used in Example 1 to investigate the influence of pH for the heat treatment (105°C) of bacterial cells. The AJ1511 strain was cultured in the Luria Broth (Difco) medium (100 ml in 500-ml Sakaguchi flask) at 30°C for 18 hours, and the cells were collected by centrifugation, washed twice with distilled water, and subjected to a heat treatment at pH 4 and 105°C for 2 hours in the same manner as that of Example 1 to obtain bacterial cells heat-treated under acidic condition. Subsequently, immunostimulating activity of these cells was investigated by the in vitro test method described above as the test method 2. The results are shown in Table 6.

**Table 6**

| | pH for heat treatment | Bacterial cell amount (mg/ml) | |
|---|---|---|---|
| | | 1 | 0.04 |
| Test group | pH 4 | 1.66 | 0.52 |
| | pH 7 | 0.22 | 0.28 |
| Control group | Without heat treatment | 0.12 | 0.21 |

| | | | |
|---|---|---|---|
| (The numerals shown in the table indicate the total IgA concentrations (µg/ml) in the culture supernatants) | | | |

It was found that, even when the washed cells were used, the immunostimulating activity of the bacterial cells heat-treated under the acidic condition (pH 4) was higher than that of the bacterial cells subjected to a heat treatment under a neutral condition or the bacterial cells not subjected to any heat treatment. This result demonstrated that the immunostimulating activity originated in the bacterial cell components, and did not originate in components derived from the medium (amino acids, saccharides, etc.) or reaction product of these with the cell components.

### Example 5: Infection prevention effect in white spot syndrome virus infection model of Japanese tiger prawn

White spot syndrome virus (henceforth abbreviated as WSSV) infection prevention effect of a sample obtained by subjecting the bacterial cells of *Corynebacterium glutamicum* as a fermentation by-product to a heat treatment (105°C, 2 hours) under a weakly acidic condition (pH 4) in the same manner as that of Example 1 (henceforth referred to as "bacterial cells heat-treated under acidic condition") was examined in Japanese tiger prawn by the test method 5 described below. As a control, feed not containing the bacterial cells heat-treated under acidic condition was used (control group).

The infectious disease caused by WSSV is a viral infectious disease globally spreading from around 1990, which has brought serious damages to shrimp and prawn raising. Also in Japan, it first broke out in 1993 in the regions where Japanese tiger prawn seeds were imported from China, and spread over the whole western part of Japan in the next year to cause serious damages to the Japanese tiger prawn raising industry. Because of the high mortality and infection rate, it is the most important infectious disease at prawn farming spots.

### (Test method 5) Infection prevention effect in Japanese tiger prawn WSSV infection model

### 5-1. Feeding method

Japanese tiger prawns (body weight, about 18 g) as the test animals were fed in 200-L tanks in a number of 40 prawns/tank. The bacterial cells heat-treated under acidic condition were added in an amount of 0.2, 0.5, or 1.0% by weight to feed materials including fish meal, minerals, vitamins and so forth, 10% by weight of water was added, and the mixture was sufficiently kneaded. Then, the kneaded mixture was palletized by using a fine disc pelleter, and the pellets were dried at 40°C for 10 hours in a heat dryer to prepare a feed for prawns containing the bacterial cells heat-treated under acidic condition. Furthermore, for a control group, feed not containing the bacterial cells heat-treated under acidic condition was prepared. These feeds were fed to the prawns of the test groups and control group from the day 7 days before the test starting day (WSSV infection day) to the 15th day after the start of the test. The daily dose for the groups corresponded to 0, 20, 50 or 100 mg/kg of body weight in terms of the bacterial cell amount.

### 5-2. Method for infecting WSSV

Cephalothoraxes of the Japanese tiger prawns died of WSSV infection (20 g) were homogenized in sterilized seawater (40 ml), and the obtained suspension was centrifuged at 4°C and 3000 rpm for 10 minutes. After the centrifugation, virus amount in the supernatant was measured by the RT mPCR method, and the viruses were suspended in seawater (20 L) at a density of 1800 copies/ml. Forty Japanese tiger prawns for each test group were immersed into this suspension for 2 hours, and thereby artificially infected with white spot syndrome viruses.

### 5-3. Evaluation of effect

The prawns were observed everyday during the test period (15 days after the infection with WSSV), and if there were died prawns, deaths were recorded. The test results are shown in Table 7.

**Table 7**

| Number of days after challenge | Control group | Bacterial cells heat-treated under acidic condition-administered group | | |
|---|---|---|---|---|
| | | 20 mg/kg body weight | 50 mg/kg body weight | 100 mg/kg body weight |
| 0 | 0 | 0 | 0 | 0 |
| 1 | 0 | 0 | 0 | 0 |
| 2 | 2 | 3 | 0 | 0 |
| 3 | 5 | 6 | 3 | 0 |
| 4 | 8 | 6 | 0 | 2 |
| 5 | 6 | 0 | 4 | 0 |
| 6 | 2 | 5 | 1 | 2 |
| 7 | 7 | 3 | 2 | 0 |
| 8 | 2 | 2 | 3 | 0 |
| 9 | 1 | 0 | 0 | 0 |
| 10 | 0 | 0 | 1 | 0 |
| 11 | 1 | 0 | 0 | 0 |
| 12 | 0 | 0 | 0 | 0 |
| 13 | 0 | 0 | 0 | 0 |
| 14 | 0 | 0 | 0 | 0 |
| 15 | 0 | 0 | 0 | 0 |
| Cumulative death (number of died prawn) | 34 | 25 | 14 | 4 |
| Survival rate (%) | 15.0 | 37.5 | 65.0 | 90.0 |

It was confirmed that the number of prawns died from WSSV infection decreased in the group administered with the bacterial cells heat-treated under acidic condition as compared to the control group, of which survival rate was 15.0%. In particular, with administration of 100 mg/kg body weight of the bacterial cells heat-treated under acidic condition, a survival rate of 90% was obtained, and thus remarkable WSSV infection prevention effect could be confirmed.

### Example 6: Infection prevention effect of Escherichia coli cells heat-treated under acidic condition in Japanese tiger prawn WSSV infection model

WSSV infection prevention effect of cells heat-treated under acidic condition of *Escherichia coli* as the enterobacterium was investigated.

WSSV infection prevention effect of a sample obtained by subjecting dry cells as a fermentation by-product of L-lysine production utilizing *Escherichia coli* to a heat treatment (105°C, 2 hours) under a weakly acidic condition (pH 4) in the same manner as that of Example 1 (henceforth referred to as *"E. coli* cells heat-treated under acidic condition") was examined in Japanese tiger prawns by the test method 6 described below. As a control, feed not containing the bacterial cells heat-treated under acidic condition was used (control group).

### (Test method 6)

A WSSV infection test was carried out in the same manner as that of the test method 5 by using the aforementioned *E. coli* cells heat-treated under acidic condition. As control group and test groups, four kinds of groups were prepared, for which doses of the *E. coli* cells heat-treated under acidic condition were 0, 20, 50, and 100 mg/kg body weight. The test results are shown in Table 8.

**Table 8**

| Number of days after challenge | Control group | *E. coli* cells heat-treated under acidic condition-administered group | | |
|---|---|---|---|---|
| | | 20 mg/kg body weight | 50 mg/kg body weight | 100 mg/kg body weight |
| 0 | 0 | 0 | 0 | 0 |
| 1 | 0 | 0 | 0 | 0 |
| 2 | 2 | 1 | 0 | 2 |
| 3 | 5 | 1 | 2 | 7 |
| 4 | 8 | 0 | 2 | 6 |
| 5 | 6 | 4 | 5 | 2 |
| 6 | 2 | 0 | 0 | 4 |
| 7 | 7 | 1 | 3 | 1 |
| 8 | 2 | 1 | 0 | 0 |
| 9 | 1 | 0 | 2 | 1 |
| 10 | 0 | 0 | 0 | 0 |
| 11 | 1 | 0 | 1 | 0 |
| 12 | 0 | 0 | 0 | 0 |
| 13 | 0 | 0 | 0 | 0 |
| 14 | 0 | 0 | 0 | 0 |
| 15 | 0 | 0 | 0 | 0 |
| Cumulative death (number of died prawn) | 34 | 8 | 15 | 23 |
| Survival rate (%) | 15.0 | 80.0 | 62.5 | 42.5 |

A marked WSSV infection prevention effect was confirmed in the group administered with 20 mg/kg body weight of the *E. coli* cells heat-treated under acidic condition, in which the survival rate was 80.0% (survival rate of the control group, 15.0%).

### Example 7: Evaluation of immunostimulating activity using phagocytic ability of Japanese tiger prawn hemocytes as index

Immunostimulating activity of a sample obtained by subjecting the bacterial cells of *Corynebacterium glutamicum* as a fermentation by-product to a heat treatment (105°C, 2 hours) under a weakly acidic condition (pH 4) in the same manner as that of Example 1 (henceforth referred to as "bacterial cells heat-treated under acidic condition") was evaluated by a test method described as the test method 7 described below by using phagocytic ability of Japanese tiger prawn hemocytes as index.

Phagocytosis is a biophylactic mechanism of the natural immunity system commonly present in a wide range of animals from higher animals such as mammals to lower animals such as invertebrates. Specifically, phagocytic cells such as macrophages and neutrophiles eliminate invading foreign substances from the inside of the body by englobing them into the cells. It is generally considered that immunostimulating substances such as peptidoglycans exhibit the immunostimulating effect by enhancing phagocytic ability of such cells, and the ability can serve as an index for evaluating immunostimulants. Japanese tiger prawns belonging to the crustaceans do not have cells called macrophage and neutrophile, and hemocytes play the same role.

### (Test method 7) Phagocytic ability of hemocytes of Japanese tiger prawn

### 7-1. Breeding of prawns

As the test animals, 15 of Japanese tiger prawns (about 18 g for each) were fed in a 60-L tank for each test group.

### 7-2. Feeding method

The bacterial cells heat-treated under acidic condition were added in an amount of 0, 0.2, 0.5, or 1.0% by weight to feed materials including fish meal, minerals, vitamins and so forth, 10% by weight of water was added, and the mixture was sufficiently kneaded. Then, the kneaded mixture was palletized by using a fine disc pelleter, and the pellets were dried at 40°C for 10 hours in a heat dryer to prepare a feed for prawns containing the bacterial cells heat-treated under acidic condition. This feed was fed to the prawns for 23 days. The daily doses of the bacterial cells heat-treated under acidic condition were 0, 20, 50 and 100 mg/kg of body weight.

### 7-3. Phagocytosis test

Blood was collected from 5 prawns for each group before the start of the feeding, 3 days and 7 days after the start of the feeding, and granulocytes were separated by Percoll density gradient centrifugation. A suspension of the cells (200 µl) was spread on cover glass placed in a plastic petri dish to adhere the hemocytes, then a yeast suspension (2 ml) was added, and the cells were cultured for 2 hours. After completion of the culture, the cells were fixed with a fixation solution, then stained with the Wright reagent, and observed under a microscope.

### 7-4. Evaluation method

Evaluation was performed on the basis of yeast phagocytosis ratio of hemocytes ((Number of hemocytes englobing yeast/Number of observed hemocytes) x 100), number of englobed yeast cells per one hemocyte (Total number of yeast cells englobed by hemocytes/Number of observed hemocytes), and phagocytic index ((Number of hemocytes englobing yeast/Number of observed hemocytes) x Number of englobed yeast cells per one hemocyte x 100). The test results are shown in Table 9.

**Table 9**

| Test item | Number of administration day | | | | | | | | |
|---|---|---|---|---|---|---|---|---|---|
| | 0 | 3 | | | | 7 | | | |
| | Dose of bacterial cells heat-treated under acidic condition (mg/kg body weight) | | | | | | | | |
| | 0 | 0 | 20 | 50 | 100 | 0 | 20 | 50 | 100 |
| Phagocytosis ratio | 12.5 ±2.51 | 10.8 ±3.11 | 13.6 ±4.03 | 18.7* ±3.07 | 29.3** ±4.06 | 13.2 ±2.75 | 15.7 ±3.64 | 34.8** ±5.04 | 45.3** ±4.75 |
| Number of englobed yeast cells per one hemocyte | 3.0 ±0.79 | 2.9 ±0.83 | 3.1 ±1.02 | 3.7 ±0.86 | 5.2 ±1.01 | 3.3 ±0.74 | 3.8 ±0.82 | 4.9* ±0.68 | 6.5** ±1.42 |
| Phagocytic index | 37.5 ±6.30 | 31.3 ±6.16 | 42.2 ±7.22 | 69.2* ±7.74 | 152.4** ±11.73 | 43.6 ±8.31 | 59.7 ±8.15 | 170.5** ±15.20 | 294.5** ±18.46 |

For all the evaluation items, phagocytosis ratio, number of englobed yeast, and phagocytic index, a tendency that immunostimulation was provided by administration of the bacterial cells heat-treated under acidic condition was observed. In particular, in the group using administration of 100 mg/kg body weight, values indicating marked immunostimulation were obtained for all the evaluation items both three days and seven days after the administration compared with the control group.

### Example 8: Phagocytic ability evaluation test for prawn hemocyte cells using E. coli cells heat-treated under acidic condition

### (Test method 8)

*E. coli* cells as a fermentation by-product were subjected to a heat treatment under an acidic condition in the same manner as that of Example 6 (henceforth referred to as *"E. coli* cells heat-treated under acidic condition"), and used for evaluation of phagocytic ability performed by a method the same as the test method 7. As the test groups, four kinds of groups were prepared, for which daily doses of the *E. coli* cells heat-treated under acidic condition were 0, 20, 50, and 100 mg/kg body weight. The test results are shown in Table 10.

**Table 10**

| Test item | Number of administration day | | | | | | | | |
|---|---|---|---|---|---|---|---|---|---|
| | 0 | 3 | | | | 7 | | | |
| | Dose of *E. coli* cells heat-treated under acidic condition (mg/kg body weight) | | | | | | | | |
| | 0 | 0 | 20 | 50 | 100 | 0 | 20 | 50 | 100 |
| Phagocytosis ratio | 12.5 ±2.51 | 10.8 ±3.11 | 19.3 ±4.49 | 17.6 ±2.25 | 20.1 ±4.03 | 13.2 ±2.75 | 39.6 ±4.23 | 27.9 ±3.86 | 18.7 ±2.53 |
| Number of englobed yeast cells per one hemocyte | 3.0 ±0.79 | 2.9 ±0.83 | 4.1 ±0.77 | 3.8 ±0.81 | 3.2 ±0.68 | 3.3 ±0.74 | 5.8 ±1.02 | 4.4 ±0.81 | 3.3 ±0.83 |
| Phagocytic index | 37.5 ±6.30 | 31.3 ±6.16 | 79.1 ±12.16 | 66.9 ±6.53 | 64.3 ±6.47 | 43.6 ±8.31 | 229.7 ±20.05 | 122.8 ±11.37 | 61.7 ±10.22 |

Values indicating marked immunity activation were obtained for all the evaluation items seven days after the administration in the test group administered with 20 mg/kg body weight of the *E. coli* cells heat-treated under acidic condition as compared to the control group.

### Example 9: Infectious disease prevention effect in bastard halibut farm

Infectious disease prevention effect of bacterial cells (bacterial cells heat-treated under acidic condition) obtained by subjecting cells of *Corynebacterium glutamicum* as a fermentation by-product (AJITEIN, Indonesia Ajinomoto Co., Inc.) to a heat treatment (105°C, 2 hours) under a weakly acidic condition (pH 4) in the same manner as that of Example 1 was confirmed by the test method 7 in a bastard halibut farm.

### (Test method 9) Field test with bastard halibuts 9-1. Bastard halibut farm for performing test

A field test was performed in a bastard halibut farm in Nagato-shi, Yamaguchi-ken, Japan over two months from July 1 to August 31, 2011. In this farm, farming is performed completely as inland culture, seawater is directly pumped up, and free-flowing water is used for feeding.

### 9-2. Fed fish

For the field test, 2000 bastard halibuts (body weight, about 80 g) were used for both the test group and the control group. As for feeding, bastard halibuts of the groups were controlled in concrete tanks having a size of 7 m square and adjacently build on land.

### 9-3. Feeding method

The bacterial cells heat-treated under acidic condition were administered by adding them to dry pellet feed for bastard halibut "HIRAMEOH" (Higashimaru Co., Ltd.). As for the addition method, water containing 0.5% by weight of guar gum was used as an adhering agent in an amount of 10% by weight of the feed to adhere the bacterial cells heat-treated under acidic condition on the feed, and the feed was given to the bastard halibuts, so that the dose of the bacterial cells heat-treated under acidic condition was 70 mg/kg body weight. As the feed for the control group, feed prepared in the same manner except that the bacterial cells heat-treated under acidic condition were not added was used.

### 9-4. Evaluation method

Number of death and cause of death were investigated over two months from the start of the administration of the bacterial cells heat-treated under acidic condition. The results of the test are shown in Table 11.

**Table 11**

| Test item | | Control group | Bacterial cells heat-treated under acidic condition-administered group |
|---|---|---|---|
| Number of fish used for test (number of fish) | | 2000 | 2000 |
| Number of death (number of fish) | | 161 | 77 |
| Mortality (%) | | 8.1 | 3.9 |
| Survival rate (%) | | 92 | 96.2 |
| Number of death by disease (number of fish, %) | | | |
| | Streptococcosis | 69 (3.5) | 8 (0.4) |
| | New type streptococcosis | 15 (0.8) | 0 (0.0) |
| | Edwardsiellosis | 47 (2.4) | 40 (0.2) |
| | Vibriosis | 20 (1.0) | 9 (0.5) |
| | Unknown | 10 (0.5) | 20 (1.0) |

It was confirmed that, in the group administered with the bacterial cells heat-treated under acidic condition, mortality due to infectious diseases was lowered to 3.9% from 8.1%. In particular, effects on streptococcosis and new type streptococcosis were remarkable.

### Industrial Applicability

By administering the immunostimulant of the present invention to mammals, fowls, fishes, crustaceans, and so forth, immune activity of these animals can be enhanced. The immunostimulant of the present invention can be produced at low cost, and is safe. Therefore, the immunostimulant of the present invention can be used for feed or as feed additive for the aforementioned animals.

## Claims

1. An immunostimulant for a mammal, fowl, fish, or crustacean, which comprises sterilized bacterial cells obtained by heat treating cells of a coryneform bacterium or an enterobacterium under a condition of pH 2 to 5.

2. The immunostimulant according to claim 1, wherein pH is 3 to 5.

3. The immunostimulant according to claim 1 or 2, wherein the heat treating is performed at 90 to 120°C for 3 minutes to 4 hours.

4. The immunostimulant according to any one of claims 1 to 3, wherein the cells of the coryneform bacterium or enterobacterium are derived from an amino acid- or nucleic acid-fermentation by-product which contains the bacterium.

5. The immunostimulant according to any one of claims 1 to 4, wherein the coryneform bacterium is *Corynebacterium glutamicum.*

6. The immunostimulant according to any one of claims 1 to 4, wherein the enterobacterium is *Escherichia coli* or *Pantoea ananatis.*

7. The immunostimulant according to any one of claims 1 to 6, wherein the coryneform bacterium or the enterobacterium is an L-amino acid or nucleic acid-producing bacterium.

8. A feed for a mammal, fowl, fish or crustacean, which contains the immunostimulant according to any one of claims 1 to 7.

9. The feed according to claim 8, which contains 0.01 to 10% by weight of the immunostimulant according to any one of claims 1 to 7.

10. A method for producing an immunostimulant for a mammal, fowl, fish, or crustacean, which comprises heat treating cells of a coryneform bacterium or an enterobacterium under a condition of pH 2 to 5, and using the obtained sterilized cells as an active ingredient.

11. A method for immunostimulation, which comprises administering the immunostimulant according to any one of claims 1 to 7 or the feed according to claim 9 or 10 to a mammal, fowl, fish, or crustacean.

12. The method according to claim 11, wherein infection of a bacterium or a virus to the mammal, fowl, fish, or crustacean is prevented by immunostimulation, and the bacterium or virus is selected from the group consisting of verotoxin-producing *Escherichia coli, Salmonella* bacterium, *Mycoplasma* bacterium, *Clostridium* bacterium, *Lawsonia* bacterium, *Streptococcus* bacterium, new type *Streptococcus* bacterium, *vibrio* bacterium, *Flexibacter maritimus,* white spot syndrome virus, and yellowhead virus.
